# EUROPEAN PATENT APPLICATION

(11) **EP 4 467 133 A1**
(43) Date of publication of application: **27.11.2024**
(21) Application number: 23743538.3
(22) Date of filing: 20.01.2023
(51) Int. Cl.: A61K 9/50, A61K 31/713, A61K 31/711, A61K 31/7105, A61K 9/19, A61P 1/00, A61K 35/20, A61P 29/00

(54) **COMPOSITION FOR IMPROVING INTESTINAL HEALTH AND FUNCTION COMPRISING MILK-DERIVED EXOSOMES AS ACTIVE INGREDIENT, AND MANUFACTURING METHOD THEREOF**

(30) Priority: 21.01.2022 KR 20220009019; 10.08.2022 KR 20220099821
(71) Applicant: Korea Institute of Science and Technology, Seoul 02792 (KR)
(72) Inventor: YANG, Yoosoo, Seoul 02792 (KR); KIM, Sun Hwa, Seoul 02792 (KR); HAN, Geonhee, Seoul 02792 (KR); JANG, Yeongji, Seoul 02792 (KR); KIM, Hyosuk, Seoul 02792 (KR); CHO, Haeun, Seoul 02792 (KR)
(74) Representative: advotec.
(86) International application number: PCT/KR2023/001034
(87) International publication number: WO 2023/140695

(57) **Abstract**

The present invention relates to a composition for improving intestinal health and function comprising: a milk-derived exosomes; and a therapeutic agent, wherein the therapeutic agent is DNA, RNA, or a small molecule, and the therapeutic agent is positioned and encapsulated in spaces inside the exosomes. In addition, provided is a method for manufacturing a composition for improving intestinal health and function, the method comprising the steps of: (a) isolating exosomes from milk; (b) forming micropores on the surfaces of the exosomes by subjecting the separated exosomes to electroporation; (c) mixing the exosomes that have undergone electroporation in step (b) with a therapeutic agent; and (d) removing therapeutic agent which has not been encapsulated inside the exosomes.

## Description

### [Technical Field]

### Cross-Reference to Related Application

The present application claims priority to Korean Patent Application No. 10-2022-0009019, filed on January 21, 2022 and Korean Patent Application No. 10-2022-0099821, filed on August 10, 2022, the entire contents of which are incorporated herein by reference.

### DESCRIPTION OF NATIONAL SUPPORT RESEARCH AND DEVELOPMENT

This study was conducted under the administration of the Korea Institute of Science and Technology (KIST) and supported by the KIST Research and Operating Expenses Support (R&D) (Major project expenses) Program funded by the Ministry of Science and ICT (Project ID: 1711152094, Hyper-connected Medical Device Reverse Translational Research Project), the KIST Research and Operating Expenses Support (Major project expenses) Program funded by the Ministry of Science and ICT (Project ID: 1711173292, Personalized Diagnosis, Treatment, Regeneration, Rehabilitation, and New Drug Development), and the Basic Technology Development Program for Innovative New Drugs funded by the Ministry of Science and ICT (Project ID: 1711160075, Development of Innovative siRNA Oral Therapeutic Agent for the Treatment of Inflammatory Bowel Disease).

The present disclosure relates to a composition for improving intestinal health function, which includes milk-derived exosomes as an active ingredient.

### [Background Art]

An exosome is a type of extracellular vesicle with a diameter of 30 to 150 nm and is secreted by various types of cells. These exosomes are beginning to be used in next-generation drug delivery technologies as it is being recognized that they play an important role in transmitting information between cells. However, the amount of exosomes secreted by cells is too small, it is difficult to achieve high purity, and they have relatively unstable properties, which limits their mass production. Recently, food-derived exosomes have been in the limelight. These food-derived exosomes may be readily available and are generally safe. Among these, milk-derived exosomes, in particular, have anti-inflammatory properties and include microRNAs in the exosomes themselves that are important for mediating their pathological and physiological activities. The milk-derived exosomes may isolate and purify exosomes from milk in high yields and maintain high stability even in the face of drastic environmental changes, like pH or temperature. In addition, the milk-derived exosomes are highly bioavailable, allowing for oral administration of drugs, especially when lesions are present in the gastrointestinal tract, such as in inflammatory bowel diseases.

### [Disclosure]

### [Technical Problem]

In one aspect, there is provided a composition for improving intestinal health function.

In one aspect, there is provided a composition for improving intestinal health function, in which a therapeutic agent is positioned and encapsulated in an internal space of an exosome.

In one aspect, there is provided a composition that is orally administerable and for improving an intestinal-related disease or function.

In one aspect, there is provided a method of manufacturing a composition for improving intestinal health function.

### [Technical Solution]

In one aspect, the present invention is directed to providing a composition for improving intestinal health function including: a milk-derived exosomes; and a therapeutic agent, in which the therapeutic agent may be DNA, RNA, or a small molecule, and the therapeutic agent may be positioned and encapsulated in spaces inside the exosomes.

In another aspect, there is provided a method of manufacturing a composition for improving intestinal health function, the method including the steps of (a) isolating exosomes from milk; (b) forming micropores on the surfaces of the exosomes by subjecting the isolated exosomes to electroporation; (c) mixing the exosomes that have undergone electroporation in step (b) with a therapeutic agent; and (d) removing the therapeutic agent which has not been encapsulated inside the exosomes.

### [Advantageous Effects]

Exosomes can be easily isolated and purified in high yield from readily available and easily accessible milk to overcome the low yield and high maintenance cost of cell-derived extracellular vesicles. In addition, instead of cell-derived extracellular vesicles, which have relatively unstable properties, a therapeutic agent can be encapsulated into milk-derived exosomes, which have the property of maintaining high stability even under drastic environmental changes in pH, temperature, and the like, using an electroporation method. These milk-derived exosomes have the advantage of being highly bioavailable, allowing for oral administration of drugs, which is particularly useful when lesions are present in the gastrointestinal tract, such as in inflammatory bowel diseases.

### [Description of Drawings]

FIG. 1a illustrates the expression levels of exosomal protein markers (Alix, Tsg-101, and CD9) and a non-exosomal protein marker (GM130).
FIG. 1b is a graph of the size distribution of milk-derived exosomes.
FIG. 1c illustrates representative TEM images of milk-derived exosomes.
FIG. 2a is confocal microscopy images of RAW264.7 and NCM460 cells cultured with 0.05 or 0.1 mg/mL of Cy5.5-labeled Col-exo for 0, 1, 4, 8, 12, or 24 hours. In the drawings, the darker small circles mean DAPI, and the lighter circles mean Col-exo.
FIG. 2b illustrates the cell proliferation rate of NCM460 and RAW264.7 cells cultured with 0, 0.1, 0.2, 0.5, or 1 mg/ml of milk-derived exosomes for 24 hours (n=6; ns = not significant, **p<0.01 and ****p<0.0001).
FIG. 2c is a graph illustrating the cell viability of NCM460.
FIG. 3a is an image confirming the reduced ROS within the cells.
FIG. 3b is a graph illustrating the expression of mRNAs encoding inflammatory cytokines in M1-type macrophages (n=4; *p<0.05, **p<0.01, ****p<0.0001).
FIGS. 4a and 4b are images of the biodistribution of Cy5.5-labeled milk-derived exosomes. FIG. 4a is an entire organ image and FIG. 4b is a cross-sectional image of the organ.
FIG. 4c is a schematic view illustrating the schedule of DSS colitis induction treatment and oral administration of milk-derived exosomes.
FIG. 4d is a graph illustrating, from left, weight loss, change in DAI index for DSS colitis-induced mice (n≥10; ns=not significant, *p<0.05, **p<0.01, and ****p<0.0001), and mice sacrificed on day 27 and colon length measured (n≥10; ns=not significant, ***p<0.001, and ****p<0.0001).
FIGS. 5a to 5c illustrate the effectiveness of Example 1 in attenuating the severity of DSS-induced colitis. FIG. 5a is a histopathologic image of colon tissue analyzed by hematoxylin and eosin (H&E) staining on day 27 (∘ loss of surface epithelium, ▲ ulceration, ↑ infiltration of inflammatory cells). FIG. 5b is a representative immunohistochemistry (IHC) image for COX-2, with arrows indicating a position where COX-2 protein is highly expressed. FIG. 5c is a representative image of colon necrosis detected by TUNEL staining.
FIG. 6a illustrates ROS in the colon measured by chemiluminescence assay (n≥7; ns>0.05, ***p<0.001 and ****p<0.0001).
FIG. 6b is a representative image of immunofluorescence (IF) staining for iNOS (blue, dark gray on the drawing: DAPI; green, light gray on the drawing: iNOS).
FIG. 6c illustrates the expression of mRNAs coding for inflammatory cytokines in colon tissue (n=4; *p<0.05 and ***p<0.001).
FIG. 7 is a graph illustrating the stability of milk-derived exosomes to an electroporation method.
FIG. 8 is an image of siRNA carried in the milk-derived exosome on an agarose gel.
FIG. 9 is a graph comparing the encapsulation efficiency of siRNAs into the milk-derived exosome according to electroporation variables.
FIGS. 10a and 10b are photographs of visualizations of milk-derived exosomes encapsulated with TNF-α siRNA and graphs illustrating relative fluorescence area sizes.
FIG. 11 is an image of intracellular uptake of milk-derived exosomes encapsulated with TNF-α siRNA (Cy3 labeled).
FIG. 12 is a confocal image and fluorescence intensity graph illustrating the intracellular delivery rate of milk-derived exosomes encapsulated with TNF-α siRNA.
FIG. 13 is a graph illustrating the gene silencing effect (in vitro) of milk-derived exosomes encapsulated with TNF-α siRNA.
FIG. 14a is a schematic view illustrating an IBD induction and treatment schedule.
FIGS. 14b and 14c are graphs of observations of weight loss and DAI index changes in IBD-induced mice.

### [Mode for Disclosure]

### Definition of terms

As used in the present specification, the term "intracellular delivery rate" means the extent to which encapsulated TNF-α siRNA is delivered into a cell, expressed as a percentage, by measuring the relative fluorescence intensity of TNF-α siRNA in the cell relative to the relative fluorescence intensity of TNF-α siRNA of cells stained with TNF-α siRNA transfected using Lipofectamin as a reference (100 %).

Hereinafter, the present invention will be described in detail.

In one aspect, the present invention is directed to providing a composition for improving intestinal health function including: a milk-derived exosomes; and a therapeutic agent, in which the therapeutic agent may be DNA, RNA, or a small molecule, and the therapeutic agent may be positioned and encapsulated in spaces inside the exosomes. In the present specification, "encapsulated by being positioned in the internal space of the exosomes" means that a substance, such as a therapeutic agent, completely enters the internal space of the exosomes and is encapsulated, and is not present only on the surface of the exosomes by methods such as attachment, adsorption, or adhesion.

In another aspect of the present invention, there is provided a method of improving intestinal health function in a subject, the method including a step of administering milk-derived exosomes and a therapeutic agent of an effective amount to a subject in need thereof, in which the therapeutic agent may be DNA, RNA, or a small molecule, and in which the therapeutic agent may be positioned and encapsulated in an internal space of the exosomes.

In another aspect of the present invention, there is provided a use of milk-derived exosomes and a therapeutic agent in manufacturing a composition for improving intestinal health function.

In another aspect of the present invention, there is provided milk-derived exosomes and a therapeutic agent for improving intestinal health function.

In one exemplary embodiment, the milk may be human, bovine, sheep, long-tailed goral, goat, camel, buffalo, or yak-derived milk, or colostrum, and is not limited thereto.

In one exemplary embodiment, the RNA may be one or more RNAs selected from the group consisting of, but not limited to, siRNA, miRNA, mRNA, shRNA, ncRNA, and antisense RNA.

In one exemplary embodiment, the siRNA may be one or more siRNAs selected from the group consisting of TNF-α siRNA, TGF-β siRNA, JAK3 siRNA, and IL-6R siRNA.

In one exemplary embodiment, the miRNA may be one or more miRNAs selected from the group consisting of Let-7b-5p, miR-320a-3p, and miR-21.

In one exemplary embodiment, the composition may be a liquid formulation and include milk-derived exosomes encapsulated with the therapeutic agent at a concentration of 100 to 1000 µg/ml. Specifically, the milk-derived exosomes have a concentration of 100 µg/ml or more, 150 µg/ml or more, 200 µg/ml or more, 250 µg/ml or more, 300 µg/ml or more, 350 µg/ml or more, 400 µg/ml or more, 450 µg/ml or more, or 500 µg/ml or more, and may have a concentration of 1000 µg/ml or less, 950 µg/ml or less, 900 µg/ml or less, 850 µg/ml or less, 800 µg/ml or less, 750 µg/ml or less, 700 µg/ml or less, 650 µg/ml or less, 600 µg/ml or less, or 550 µg/ml or less.

In one exemplary embodiment, the composition may be a lyophilized formulation and may include the milk-derived exosomes encapsulated with the therapeutic agent of 0.001 to 100 wt% based on the total weight of the composition. Specifically, the content may be adjusted by those skilled in the art depending on the concentration or type of solvent of the milk-derived exosomes encapsulated with the therapeutic agent. For example, the content may be 0.001 wt% or more, 0.01 wt% or more, 0.1 wt% or more, 1 wt% or more, 5 wt% or more, 10 wt% or more, 15 wt% or more, 20 wt% or more, 25 wt% or more, 30 wt% or more, 35 wt% or more, 40 wt% or more, 45 wt% or more, or 50 wt% or more, and may be 100 wt% or less, 95 wt% or less, 90 wt% or less, 85 wt% or less, 80 wt% or less, 75 wt% or less, 70 wt% or less, 65 wt% or less, 60 wt% or less, or 55 wt% or less.

In one exemplary embodiment, the composition may be administered orally.

In another aspect, there is provided a method of manufacturing a composition for improving intestinal health function, the method including the steps of (a) isolating exosomes from milk; (b) forming micropores on the surfaces of the exosomes by subjecting the isolated exosomes to electroporation; (c) mixing the exosomes that have undergone electroporation in step (b) with a therapeutic agent; and (d) removing the therapeutic agent which has not been encapsulated inside the exosomes.

In one exemplary embodiment, the electroporation of step (b) may be to apply a voltage of 250 to 350 V. Specifically, the voltage may be 250 V or more, 255 V or more, 260 V or more, 265 V or more, 270 V or more, 275 V or more, 280 V or more, 285 V or more, 290 V or more, 295 V or more, or 300 V or more, and may be 350 V or less, 345 V or less, 340 V or less, 335 V or less, 330 V or less, 325 V or less, 320 V or less, 315 V or less, 310 V or less, or 305 V or less.

In one exemplary embodiment, the electroporation of step (b) may be to apply 3 to 7 pulses. Specifically, the pulses may be 3 or more, 4 or more, 5 or more, and may be 7 or less, or 6 or less.

In one exemplary embodiment, the electroporation in step (b) may be to apply a pulse for a time period of 20 to 40 ms. Specifically, the pulse may be 20 ms or more, 21 ms or more, 22 ms or more, 23 ms or more, 24 ms or more, 25 ms or more, 26 ms or more, 27 ms or more, 28 ms or more, 29 ms or more, or 30 ms or more, and may be 40 ms or less, 39 ms or less, 38 ms or less, 37 ms or less, 36 ms or less, 35 ms or less, 34 ms or less, 33 ms or less, 32 ms or less, or 31 ms or less.

In one exemplary embodiment, the milk in step (a) may be human, bovine, sheep, long-tailed goral, goat, camel, buffalo, or yak-derived milk, or may be colostrum.

In one exemplary implementation, the therapeutic agent in step (c) may be DNA, and step (d) may be adding a DNase to remove the unencapsulated therapeutic agent.

In one exemplary embodiment, the therapeutic agent in step (c) may be RNA, and step (d) may be adding an RNase to remove the unencapsulated therapeutic agent.

In one exemplary embodiment, the RNA in step (c) may be one or more RNAs selected from the group consisting of siRNA, miRNA, mRNA, iRNA, ncRNA, and antisense RNA.

In one exemplary embodiment, the siRNA in step (c) may be one or more siRNAs selected from the group consisting of TNF-α siRNA, TGF-β siRNA, JAK3 siRNA, and IL-6R siRNA.

In one exemplary embodiment, the miRNA in step (c) may be one or more miRNAs selected from the group consisting of Let-7b-5p, miR-320a-3p, and miR-21.

Hereinafter, the present invention will be described in more detail with reference to exemplary embodiments. The embodiments are merely intended to illustratively describe the present invention, and the scope of the present invention is not limited by the embodiments. Any configuration, which is substantially identical in configuration and operational effect to the technical spirit disclosed in the claims of the present invention, belongs to the technical scope of the present invention.

### Example

### 1. Confirmation of effectiveness of bovine colostrum-derived exosomes in preventing and treating inflammatory bowel disease

### (1) Cell cultures

NCM460 cells and RAW264.7 cells (40071, Korean Cell Line Bank) were grown in Dulbecco's modified eagle medium (DMEM) with high glucose (SH30243.01, Hyclone, USA) containing 10% fetal bovine serum (FBS, FP-0500-A, Atlas Biologicals, USA) and 1% antibiotics-antimycotics (15240096, ThermoFisher Scientific, USA). Cells were incubated in a 37°C incubator with 5% carbon dioxide (CO₂). To induce M1-like macrophages, RAW264.7 cells were stimulated with 100 ng/ml LPS (L4524, Sigma, USA) and 20 ng/ml IFN-γ (315-05, PeproTech, UK) for 24 hours.

### (2) Isolation of exosomes from colostrum

The exosomes were prepared by continuous centrifugation at 4 °C. The bovine colostrum (obtained from Lee Sung-young Changbuk Ranch, 1-ro 20, Bongsangwandong, Gyeongsung-myeon, Changnyeong-gun, Gyeongnam, Korea) was centrifuged at 5,000 × g for 30 min and 12,000 × g for one hour using a high-speed centrifuge with a JA-14 rotor (Avanti J-E, Beckman Coulter, USA). The supernatant was passed through a 40 µm strainer (93040, SPL, Korea) and stored at -80 °C until use. The filtered milk was centrifuged through an ultracentrifuge with a 45 Ti rotor (Optima XE-100, Beckman Coulter, USA) at 35,000x g for one hour and 70,000x g for three hours at maximum acceleration/decal. The solution was separated into three layers, and the middle layer was sequentially filtered through syringe filters with pore sizes of 0.8, 0.45, and 0.2 µm (16592-K, 16555-K, 16534-K, Sartorius Stedim Biotech, Germany). The pellets considered to be exosomes were obtained by centrifugation at 100,000x g for one hour. The exosomes were resuspended in Dulbecco's phosphate-buffered saline (DPBS, LB001, Welgene, Korea) and spun overnight at 4 °C. The exosomes thus obtained are hereinafter referred to as bovine colostrum-derived exosomes (Col-exo, Example 1).

To prepare exosome-free milk (Exo-free milk, Comparative Example 1), the clear solution excluding the exosome pellet was centrifuged at 200,000x g for three hours to remove the remaining exosomes and the supernatant was considered as exosome-free milk.

### (3) Western blot

The concentration of exosome proteins was quantified using the BCA assay kit (23225, Thermofisher Scientific, USA), and the proteins were denatured with SDS-PAGE loading buffer. The samples were electrophoresed on sodium dodecyl sulfate (SDS) polyacrylamide gels. The separated proteins were transferred to a nitrocellulose membrane and the membrane was blocked with 5% skim milk. The primary antibody was bound to a target protein for 24 hours. The membrane was rinsed with tris-buffered saline, 0.1% Tween 20 detergent (TBST), and the proteins were indicated as incubated with HRP-labeled secondary antibody for 40 minutes at room temperature. The indicated proteins were visualized with Clarity Western ECL substrate (1705061, Bio-Rad, USA).

The antibodies used were Alix (1:500, NB100-65678, Novus, USA), Tsg-101 (1:1000, ab83, Abcam, USA), CD9 (1:1000, NB500-494, Novus, USA), GM130 (1:500, PA1077, Thermofisher Scientific, USA), goat anti-mouse IgG antibody (HRP) (1:1000, GTX21311-01, GeneTex, USA), and goat anti-rabbit IgG antibody (HRP) (1: 1000, GTX21310-01, GeneTex, USA).

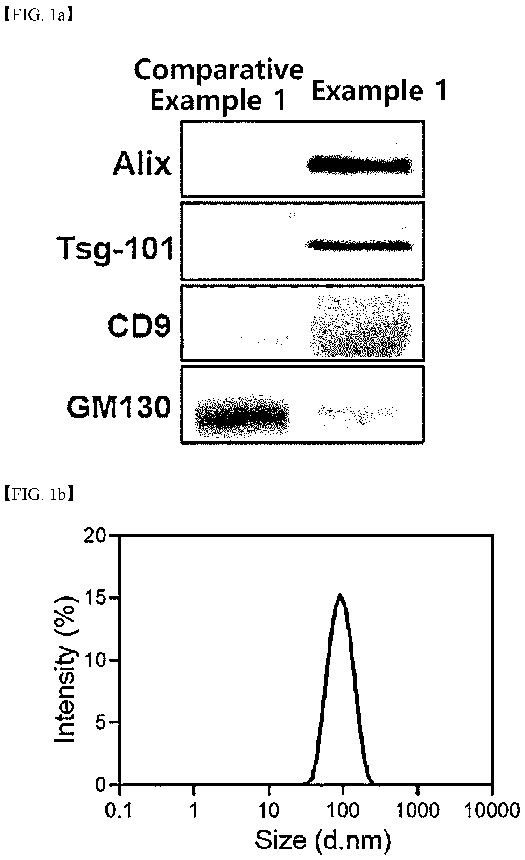

With the results for Example 1 and Comparative Example 1, as illustrated in FIG. 1a, Alix, Tsg-101, and CD9, which are markers of exosomes proteins, were identified in Example 1. In contrast, GM130, which is a non-exosomes protein, was found in Comparative Example 1. These results indicate that the presence or absence of exosomes distinguishes Comparative Example 1 (exo-free milk) and Example 1 (col-exo).

### (4) Dynamic lighting scattering (DLS) and Transmission electron microscopy (TEM)

For DLS, the exosomes were diluted to 0.1 µg/µl with distilled water and transferred to disposable cuvettes (art.01960-00, Kartell LABWARE, Italy). The size distribution of exosomes was measured by DLS (Zetasizer Nano ZS, Malvern Instruments, UK) at a fixed angle of 173°. For each sample, three independent measurements were performed and averaged for analysis by the software.

For confirmation of the TEM images, 10 µg of exosomes were deposited on a carbon-coated copper grid (CF200-Cu, Emgrid Australia, Australia) for 30 minutes at room temperature. For negative staining of exosomes, the copper grid was contrasted with uranyl acetate for one minute. The morphology of exosomes was visualized by TEM (FEI Tecnai F20 G2, Philips, Netherlands).

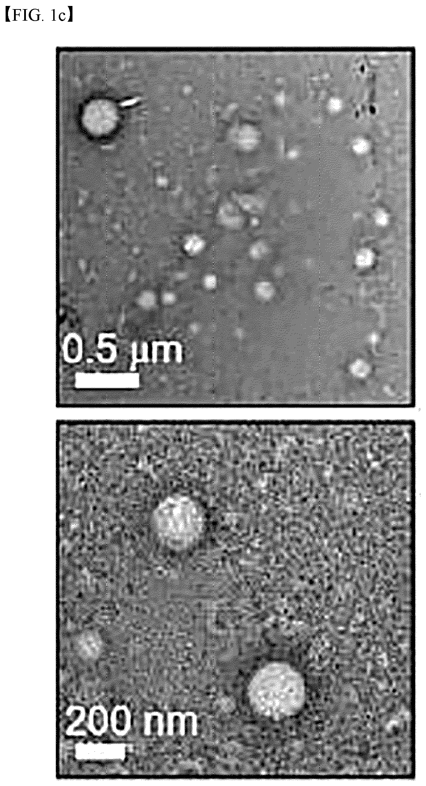

As illustrated in FIG. 1b and FIG. 1c, the exosomes of Example 1 had an average diameter of 85.2 nm and were found to be spherical in shape.

### (5) Cellular uptake of exosomes

The exosomes were labeled with 650 nM Cyanine 5.5 NHS ester by spinning overnight at 4 °C. The unbonded fragments were cleaned twice using an Airfuge. The cells were plated on confocal dishes at a density of 2 × 10⁵ cells/dish for RAW264.7 cells and 3 × 10⁵ cells/dish for NCM460 cells. The exosomes were treated with each concentration (0.05 and 0.1 mg/ml) and washed with DPBS at each point in time. To capture images, the cells were fixed with formaldehyde and then 4',6-diamidino-2-phenylindole (DAPI) was added to stain the nucleic acids for 15 minutes. The images were taken with a confocal microscope (Leica TCS SP6, Leica, Germany).

### (6) Cell proliferation and viability analysis

Cell viability assays were performed according to the Cell counting Kit-8 (CCK-8) (CK04, Dojindo, Japan) guide. The NCM460 and RAW264.7 cells were seeded in 96-well plates at a density of 6,000 and 8,000 cells/well, respectively, and allowed to be attached for 24 hours. The cells were treated with various concentrations of Comparative Example 1 (exosome-free milk) or the exosomes of Example 1 (0.5mg/ml in viability assay) for 24 hours. As an additional step for viability analysis, the cells were incubated with 2.5% DSS (0216011080, MP Biomedicals, USA) solution (w/v) for 24 hours. The medium was replaced with serum-free medium containing 10% CCK-8 solution. Absorbance at a wavelength of 450 nm was measured with a microplate reader (SpectraMAX 340, Molecular Devices, USA). The RAW264.7 cells were incubated with 100 ng/ml LPS and 20 ng/ml INF-γ for 24 hours before treatment with exosomes.

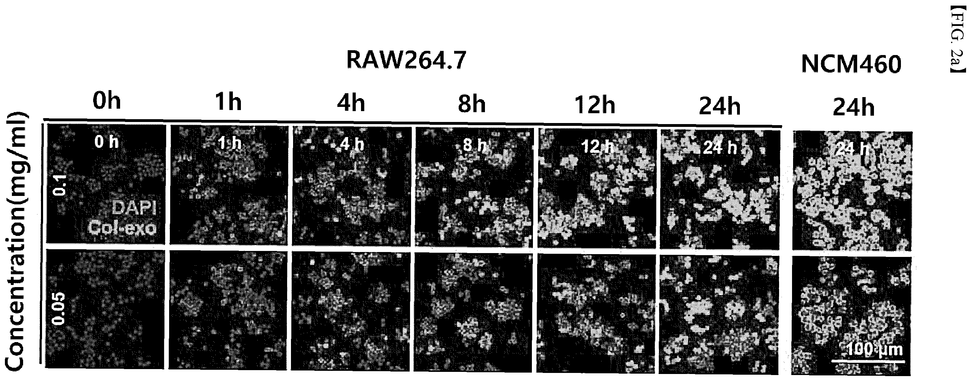

The intracellular delivery efficiency of Example 1 (Col-exo) was investigated. The RAW264.7 cells were incubated with Example 1 labeled with Cy5.5 at a concentration of 0.05 or 0.1 mg/ml for 0, 1, 4, 8, 12, or 24 hours (FIG. 2a). In FIG. 2a, the darker small circles mean DAPI, and the lighter circles mean Col-exo. The confocal images show that significant intracellular uptake of Example 1 (Col-exo) occurred within 8 hours, and the images show that the uptake saturated within 24 hours. In addition, the NCM460 cells showed a similar pattern. The uptake time of Example 1 (Col-exo) was set to 24 hours in the subsequent in vitro and in vivo experiments on the basis of these data.

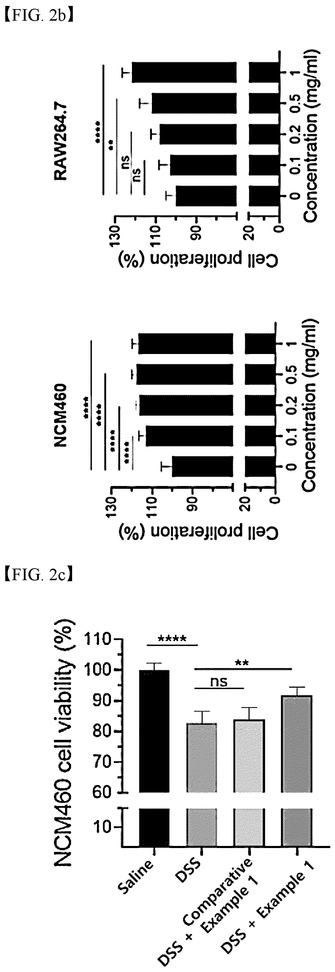

Next, to investigate the relationship between Example 1 (Col-exo) and cell growth, cell proliferation assays were performed on the NCM460 cells and RAW264.7 cells (FIG. 2b). It can be seen that when the NCM460 cells were treated with Example 1 (Col-exo) at a concentration of 0.1 mg/ml, the cell proliferation significantly increased to 113.1 ± 3.4 %. This cell proliferation remained similar up to the concentration of 1 mg/ml in Example 1. Another cell line, RAW264.7, showed a significant increase in cell growth at a concentration of 0.5 mg/ml, especially increased to 121.8 ± 4.7 % at a concentration of 1 mg/ml.

In addition, the ability of Example 1 (Col-exo) to repair external damage after damaging colon epithelial cells with dextran sulfate sodium (DSS, 36,000 - 50,000 Da), which is a chemical that causes inflammatory bowel disease (IBD), was observed (FIG. 2c). The cell damage was caused by treatment with 2.5% DSS for 24 hours, resulting in a decrease in cell growth rate to 82.7 ± 3.7 %. The group treated with Example 1 (Exo-free milk) showed similar levels of cell viability to the DSS treatment, in contrast, Example 1 (Col-exo) was able to restore cell viability to 91.8 ± 2.6 %. As a result, Example 1 (Col-exo) was found to increase the proliferation of NCM460 and RAW264.7 cells.

### (7) Confirmation of anti-inflammatory effectiveness

Next, it was confirmed whether bovine colostrum-derived exosomes were able to reduce intracellular reactive oxygen species (ROS) concentrations using the DCF-DA assay. The RAW264.7 cells were seeded at a density of 2 × 10⁵ cells in confocal dishes and treated with 100 ng/ml LPS and 20 ng/ml IFN-γ for 24 hours to induce M1 macrophages. The NCM460 cells were cultured for 24 hours at a density of 3 × 10⁵ cells/well. Each cell was treated with the exosome-free milk or exosomes at a concentration of 0.1 mg/ml for 24 hours. Oxidative stress was induced by providing 100 µM of H₂O₂ for 30 minutes and incubating the cells with 50 µM of 2',7'-dichlorofluorescin diacetate (DCFDA) for 30 minutes. After washing the cells with DPBS, fluorescence images were obtained using a confocal microscope.

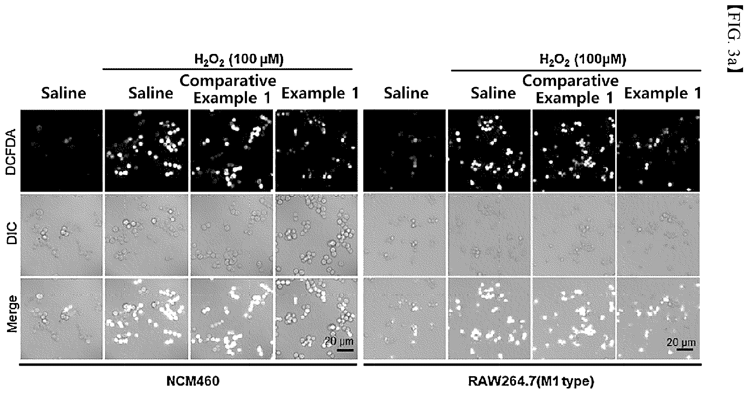

As illustrated in FIG. 3a, in both NCM460 cells and RAW264.7 cells, the intracellular ROS levels (light circles) in the Example 1 (Col-exo) treatment group were significantly reduced compared to the hydrogen peroxide treatment group or the Comparative Example 1 (Exo-free milk) treatment group.

### (8) Quantification of cytokine levels in vitro

In addition, the expression levels of mRNAs encoding intracellular cytokines involved in the inflammatory response were analyzed. The RAW264.7 cells (2.5 × 10⁵ cells/ml) were plated in culture dishes and stimulated with 100 ng/ml LPS and 20 ng/ml IFN-γ for 24 hours. The M1-induced cells were treated with exosome-free milk or exosomes for 24 hours and then washed twice with DPBS. Total RNA was extracted with TrizolTM reagent according to the manufacturer's guidelines and cDNA was generated with oligo(dT) primers after reverse transcription. Quantification of cytokines was performed by quantitative real-time PCR (qRT-PCR) using SYBR Green qPCR premix (RT501S, enzynomics, Korea) and respective primers (Table 1), and the PCR cycles were set according to the premix protocol.

**[Table 1]**

| | |
|---|---|
| Gene | Primer sequence |
| | (F: Forward; R:Reverse) |
| GAPDH | F:CATGCCGCCTGGAAACCTGCCA |
| | R: TGGGCTGGGTGGTCCAGGGGTTTC |
| TNF-α | F: AGTGGAGGAGCAGCTGGAGT |
| | R: TCCCAGCATCTTGTGTTTCTG |
| IL-6 | F:GAGGATACCACTCCCAACAGACC |
| | R: TTCACAGAGGATACCACTCC |
| IL-10 | F:CTATGCAGTTGATGAAGATGTCAAA |
| | R: CCTGGTAGAAGTGATGCCCCAGGCA |

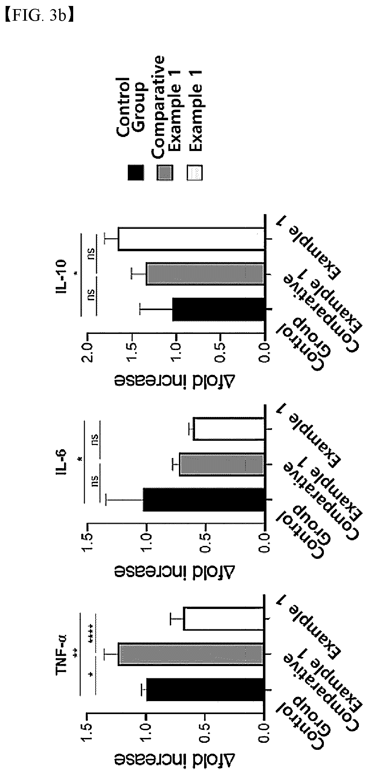

First, the RAW264.7 cells were differentiated into M1 macrophages and treated with Example 1 (Col-exo) and Comparative Example 1 (Exo-free milk), respectively. As illustrated in FIG. 3b, quantitative real-time PCR (qRT-PCR) data indicated that the mRNA expression levels of tumor necrosis factor-α (TNF-α) and interleukin-6 (IL-6) were reduced by 0.69-fold and 0.61-fold, respectively, in Example 1 compared to a control group. In contrast, the mRNA expression level of interleukin-10 (IL-10), which is an anti-inflammatory cytokine, increased 1.7-fold in Example 1 (Col-exo) group compared to the control group. Therefore, it was found that Example 1 has anti-inflammatory effects at the cellular level.

### (9) Ex vivo bio-distribution of colostrum-derived exosomes

Before confirming the effect of Example 1 (Col-exo) in a mouse model of colitis, the relative bio-distribution in organs was measured after oral administration of Cy5.5-labeled Example 1 (Col-exo). BALB/c mice were orally administered exosomes labeled with Cy5.5. Mice were subjected to oral gastric tube feeding, and 5 hours and 24 hours later, gastrointestinal tracts and other organs including heart, lungs, spleen, kidneys, and liver were collected. The fluorescence intensity of the separated organs was estimated using an in vivo imaging system (IVIS) (Lumina Series III PerkinElmer, USA). The uptake of exosomes was observed in formalin-fixed, paraffin-embedded gastrointestinal organs. The paraffin-embedded tissue was cut into 5 µm thick sections. Representative images were captured using a confocal microscope.

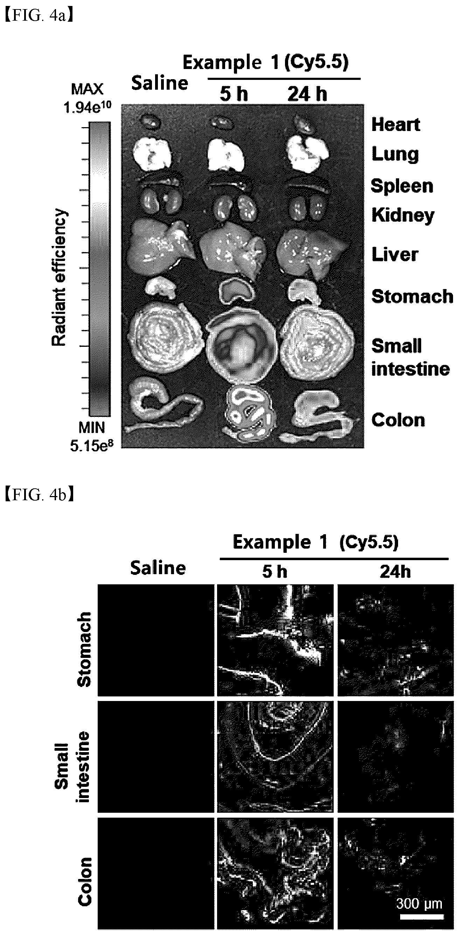

As a result, as illustrated in FIG. 4a, when the fluorescence intensity was measured 5 hours after feeding Cy5.5-labeled Col-exo, it showed a strong signal in the gastrointestinal tract, especially in the colon. In addition, it was confirmed that the fluorescent signal was significantly maintained in the stomach and colon at 24-hour point in time. Meanwhile, as can be seen in FIG. 4b, the stomach, small intestine, and colon were sliced, respectively, and the uptake of Example 1 (Col-exo) labeled with Cy5.5 into the organs was observed. The uptake of Example 1 (Col-exo) labeled with Cy5.5 was mostly found in the stomach and colon. After 24 hours, it was confirmed that uptake exosomes were significantly reduced. Therefore, Example 1 was administered orally to mice daily.

### (10) Estimation of inflammatory response in mouse models of colitis

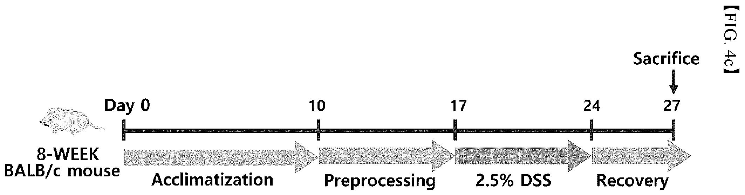

Eight-week-old BALB/c female mice (Orient Bio, Korea) were used for the ulcerative colitis model. Mice were randomly separated into three groups, healthy control (saline), DSS-induced colitis (DSS), and Col-exo pretreatment (Example 1), and subjected to a 10-day acclimatization period. Example 1 (Col-exo) group was pretreated with 1 mg of colostrum-derived exosomes daily by oral gastric tube feeding for one week, while the other groups were given saline only. Inflammatory bowel disease (IBD) was chemically induced by administering a 2.5% DSS solution in drinking water for one week at random. On day 27, mice were sacrificed and colon lengths were estimated (FIG. 4c).

### 1) Disease Activity Index (DAI)

Disease activity index was assessed by three independent observers for each mouse daily from day 18 to day 27 for weight loss, fecal viscosity, and characteristics of fecal bleeding (Table 2). The total score ranges from 0 to 12, with higher scores indicating more severe disease.

**[Table 2]**

| Score | Weight loss (%) | Fecal viscosity | Fecal bleeding |
|---|---|---|---|
| 0 | <5% | Normal | Normal |
| 1 | 5-10% | | |
| 2 | 10-15% | Runny | Occult blood |
| 3 | 15-20% | | |
| 4 | >20% | Diarrhea | Bleeding |

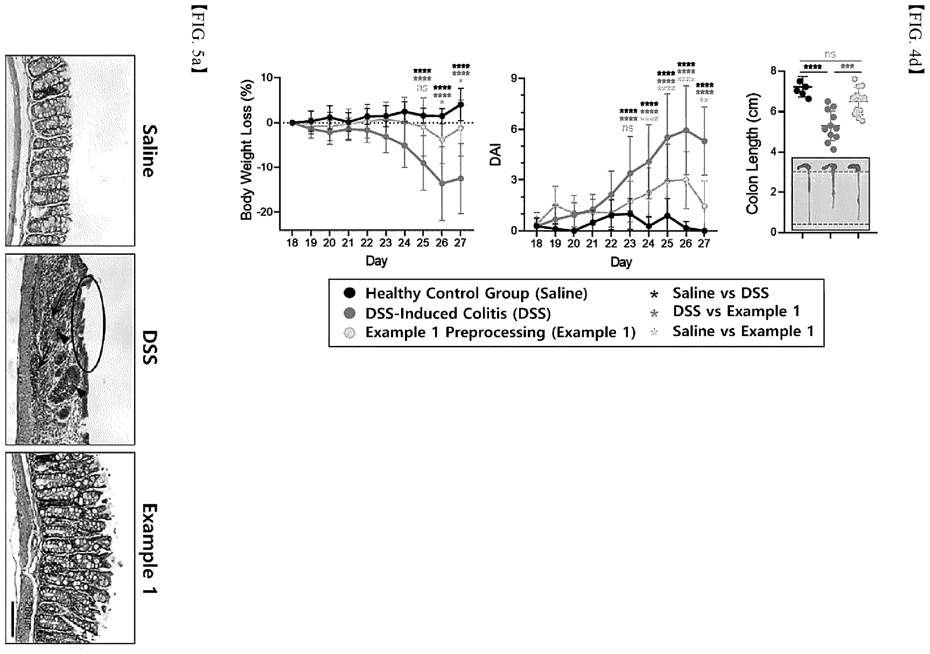

Animal experiments were conducted according to the established schedule, and the results showed that Example 1 (Col-exo) group effectively inhibited the weight loss of DSS-induced colitis mice, in contrast to the DSS group, which showed rapid weight loss. (FIG. 4d). 2) Ex vivo ROS imaging

On day 27, L-012 (120-04891, Fujifilm Wako Chemicals, Japan) was prepared by dissolution in DPBS and stored in the dark compartment at room temperature. The L-012 solution was injected intraperitoneally at a dose of 20 mg/kg and the colons of the mice were separated 15 minutes later. ROS in the separated colon was imaged by an in vivo imaging system (IVIS) (Lumina Series III PerkinElmer, USA). The data shows the average radiation efficiency for a 5-minute measurement (p/s/cm2/sr).

Animal experiments were conducted according to the established schedule, and the results showed that Example 1 (Col-exo) group effectively inhibited the weight loss of DSS-induced colitis mice, in contrast to the DSS group, which showed rapid weight loss. In addition, the disease activity index (DAI) score of Example 1 (Col-exo) group was significantly lower than that of the DSS group. Colon length in Example 1 (Col-exo) group was significantly longer than in the DSS group and recovered to a level similar to the saline group (FIG. 4d). With the experiments above, it was confirmed that Example 1 alleviates DSS-induced colitis.

### (11) Histologic analysis

The separated mouse colon tissue was prepared by Swiss-roll technique. Colon samples were immersion-fixed in formalin overnight, dehydrated, and embedded in paraffin. All tissue specimens were cut into 5 µm thick sections.

### 1) H&E (Hematoxylin and eosin)

The sections were dewaxed in xylene and stained with hematoxylin. Nuclear staining with blueing agent has been completed after washing with tap water. Proteins were nonspecifically stained by eosin (ab245880, Abcam, USA). The slides were mounted in toluene and observed using an optical microscope (BX51, Olympus Corporation, Japan).

As a result, severe superficial epithelial damage, ulceration, and infiltration of immune cells were found in the colon of the DSS group (FIG. 5a). In contrast, Example 1 group showed minor epithelial proliferation.

### 2) Immunohistochemistry (IHC)

Because COX-2 is a key molecule in the initiation of inflammation, immunohistochemistry (IHC) was used to examine the expression levels of COX-2 protein in each group. The expression of COX-2 in colon tissues was detected using Mouse and Rabbit Specific HRP/DAB (ABC) Detection IHC kit (ab64264, Abcam, USA) according to the manufacturer's protocol. The target protein was tagged with COX-2 antibody (1:500, ab15191, Abcam, USA) in PBS containing 1% BSA and 0.5% Triton X-100. Tissue sections were stained with DAB substrate and counterstained with hematoxylin. The images were captured with an optical microscope.

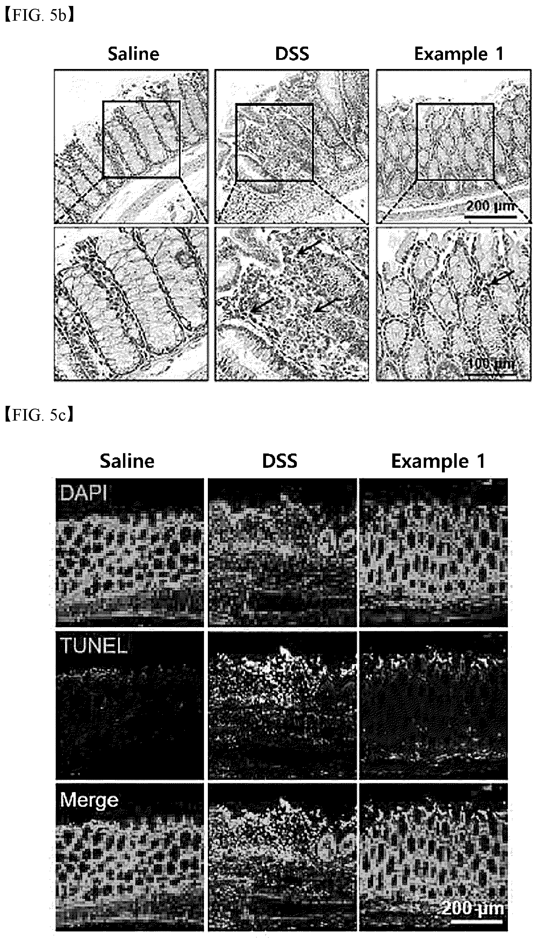

As can be seen in FIG. 5b, the expression of COX-2 protein was reduced in the colon of Example 1 (Col-exo) group compared to the DSS group.

### 3) Immunofluorescent (IF)

To visualize the extent of tissue cell death, TUNEL assays were performed according to the manufacturer's instructions for the fluorometric TUNEL System (G3250, Promega, USA). That is, rehydrated tissue sections were fixed with formaldehyde and a proteinase K solution was added for permeabilization. Formaldehyde fixation was repeated and incubation was performed with equilibration buffer. Apoptosis areas were marked by terminal deoxynucleotidyl transferase (TdT) reaction and counterstained with DAPI.

As illustrated in FIG. 5c, the cell death of colon cells occurred severely in the DSS group, as indicated by the TUNEL staining data, and was predominantly recovered in Example 1 (Col-exo) group. As a result, Example 1 was effective in reducing the severity of colitis and cell death.

### (12) Quantification of in vivo cytokine levels

To confirm the effect of Col-exo on alleviating colonic inflammation, ROS levels in the colon were measured using a luminol-based chemiluminescent probe. The mouse colon tissue was homogenized using a homogenizer (HG-15D, Wise Tis, Korea) with TrizolTM reagent. After homogenization, total RNA from colon tissue was obtained according to the procedure manual of TrizolTM reagent. After cDNA synthesis, qRT-PCR was performed using SYBR Green (RT501M, Enzynomics, Korea) according to the manufacturer's protocol. All qRT-PCR was performed using a QuantStudioTM1 Real-Time PCR instrument (Applied Biosystems, USA). Each primer is summarized in (Table 1).

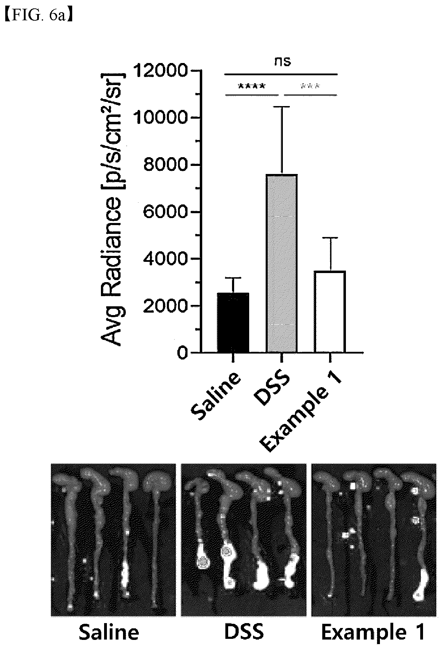

As a result, ROS levels were found to significantly increase in the DSS group and decreased by less than half in Example 1 (Col-exo) group (FIG. 6a).

### (13) Confirmation of intestinal inflammation improvement

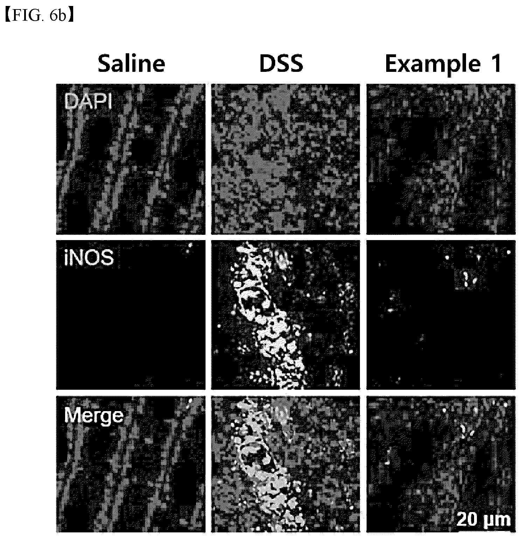

Next, to confirm the effect of Col-exo on improving intestinal inflammation, the expression of iNOS protein, which is a marker of pro-inflammatory M1-type macrophages, was confirmed using immunofluorescence (IF). Macrophage infiltration in mouse colon tissue was detected by staining for iNOS. Deparaffinized tissues were rehydrated and boiled in an antigen retrieval buffer (Citrate buffer pH 6.0) (ab93678, Abcam, USA). The slides were washed with PBS, 0.05% Tween 20 detergent (PBST) and blocked with PBST containing 5% BSA (w/v) for 10 minutes at room temperature. Alexa fluor 488 conjugated iNOS monoclonal antibody (53-5920-82, eBiosience, USA) prepared in PBS containing 1% BSA (w/v) and 0.5% Triton-100 (v/v) was used to stain sections overnight at 4 °C. Nucleic acids were stained with DAPI for 15 minutes at room temperature. The slides were mounted with water-soluble mounting solution (0100-01, Southern Biotech, USA), which was photographed under a confocal microscope. As a result, as illustrated in FIG. 6b, the iNOS protein was overexpressed in the DSS group, while it was barely observed in Example 1 (Col-exo) group.

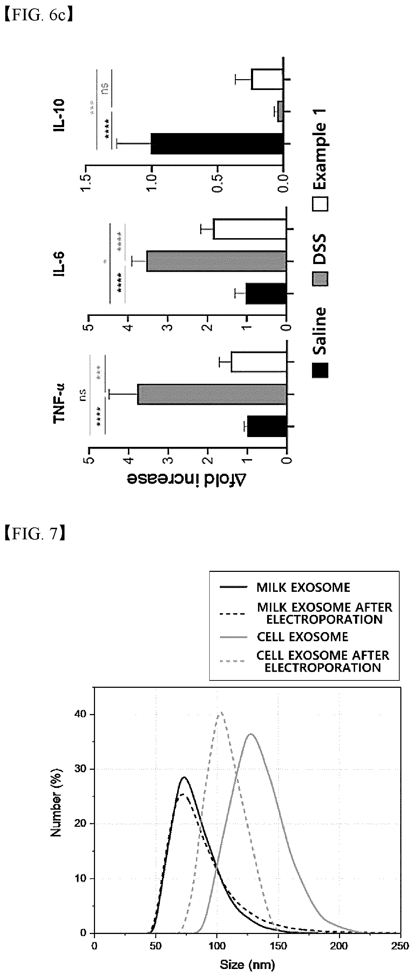

In addition, the mRNA expression of inflammatory cytokines was examined using qRT-PCR (FIG. 6c). The results of qRT-PCR showed that the mRNA levels of TNF-α and IL-6 increased 3.8-fold and 3.6-fold, respectively, in the DSS group compared to the saline group. In contrast, their mRNA expression levels were found to be similar to those of the saline group. However, the qRT-PCR results of anti-inflammatory cytokines showed the opposite trend, with IL-10 mRNA expression in the DSS group decreasing by approximately 20-fold compared to the saline group. Notably, IL-10 expression levels in Example 1 (Col-exo) treatment group recovered slightly to approximately a level of 5 times that of the DSS treatment group. These results indicate that Example 1 reduces excessive intestinal inflammatory response.

### (14) Statistical analysis

Statistical analysis of the data was performed using Prism 8.0 with parametric one-way ANOVA analysis and two-way ANOVA analysis. All results were expressed as mean ± standard deviation. All graphs were constituted in Prism 8.0 (GraphPad Software Inc, USA).

### 2. Therapeutic effect of milk-derived exosomes encapsulated with RNA on inflammatory bowel diseases

### (1) Extraction of exosomes

Commercial pasteurized milk was used, and the extraction of exosomes was performed using the same method as described in Example 1.(2).

### (2) Confirmation of stability of exosomes to electroporation

0.1mg/ml of cell-derived exosomes (HEK exo) and milk-derived exosomes (Milk exo) were subjected to an electrical shock of 300V, 30ms, 5pulses, and 1s interval, respectively, and incubated at 4 °C for three hours. The same method as the dynamic light scattering (DLS) experiment in Example 1.(4) was used to observe the size change of exosomes before and after the electrical shock.

As a result, as illustrated in FIG. 7, it was confirmed that the cell-derived exosomes became smaller in size after electroporation, while the milk-derived exosomes showed little change in the size distribution of exosomes before and after electroporation.

### (3) Encapsulation of siRNA

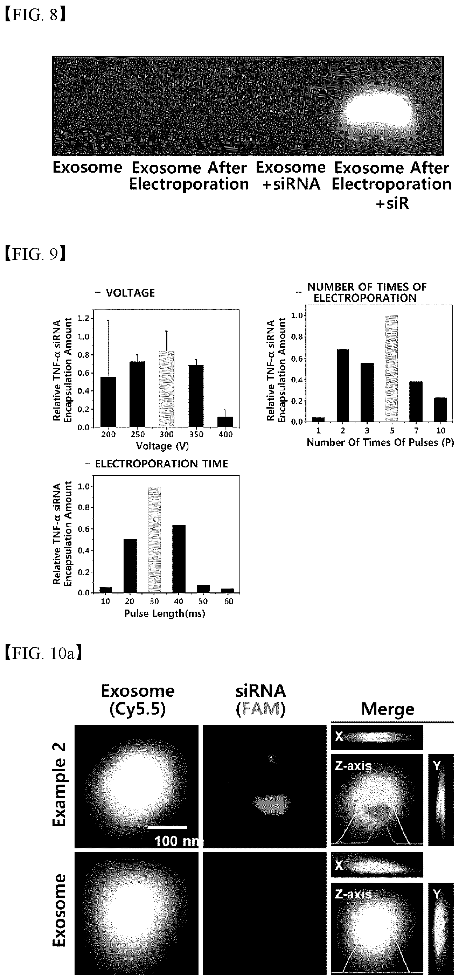

After creating microscopic pores in the exosomes by the electroporation, TNF-α siRNA was mixed into the exosome solution to encapsulate the TNF-α siRNA inside the exosomes, and then RNase was used to remove the siRNA that was not encapsulated inside the exosomes. Exosomes that were not subjected to electroporation, exosomes that were subjected to electroporation, the resultant mixture of TNF-α siRNA in exosomes that were not subj ected to electroporation, and exosomes encapsulated with TNF-α siRNA that is mixed in exosomes that were subjected to electroporation were loaded onto 1% agarose gels and electrophoresed in an electrophoresis apparatus at 100 V for 10 minutes. Then, visual confirmation was performed with a gel image analyzer. As a result, it was confirmed that the TNF-α siRNA was efficiently encapsulated inside the exosomes when the exosomes were subjected to electroporation, as illustrated in FIG. 8. Hereinafter, the exosomes encapsulated with TNF-α siRNA will be referred to as Example 2 and the exosomes not encapsulated with TNF-α siRNA will be referred to as Comparative Example 2.

### (4) Comparison of encapsulation efficiency of siRNAs according to electroporation variables

To confirm a voltage condition, 5 times of electroporation and an electroporation time of 30 ms were fixed for 0.1 mg/ml of milk-derived exosomes, and voltages of 200, 250, 300, 350, and 400 V were applied to compare the encapsulation efficiency of TNF-α siRNA, to confirm the number of times of electroporation condition, a voltage of 300 V and an electroporation time of 30 ms was fixed, and the encapsulation efficiency of TNF-α siRNA was compared when 1, 2, 3, 4, and 5 times of electroporation were applied, and to confirm an electroporation time condition, a voltage of 300 V and 5 times of electroporation were fixed, and the encapsulation efficiency of TNF-α siRNA was compared when the electrical shock was applied for a time of 10, 20, 30, 40, 50, and 60 ms. When the electroporation was applied at 300 V, 5 times, for 30 ms, it was confirmed that TNF-α siRNA was most efficiently encapsulated into exosomes (FIG. 9).

### (5) Visualization of milk-derived exosomes encapsulated with TNF-α siRNA

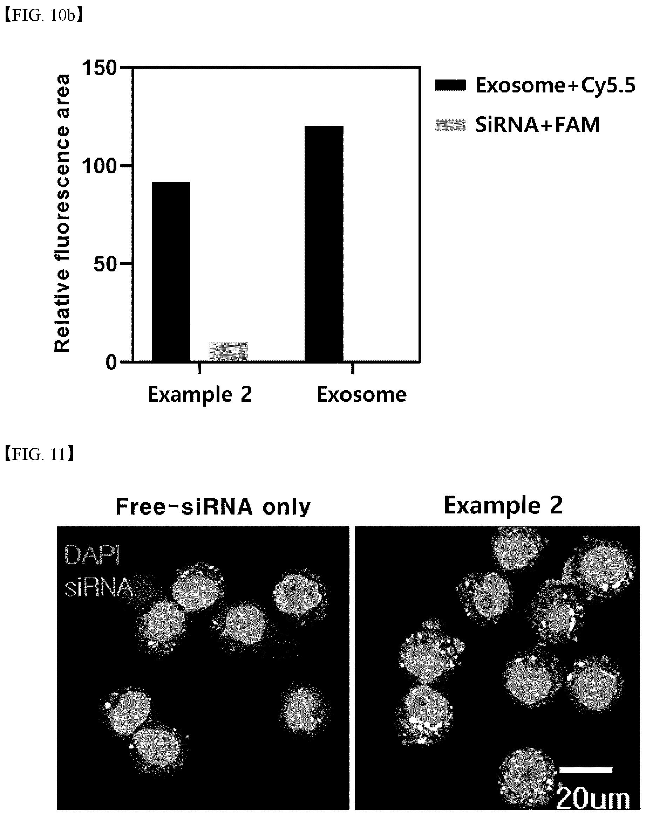

Milk-derived exosomes were subjected to electrical shocks of 300 V, 30 ms, 5 pulses, and 1 s interval, respectively, and then encapsulated with TNF-α siRNA (FAM labeled) and incubated for three hours, and the milk-derived exosomes were labeled with Cy5.5. The milk-derived exosomes encapsulated with TNF-α siRNA (Example 2) were mixed with Fluoromount-G solution and fixed on slide glasses. Then, the milk-derived exosomes encapsulated with TNF-α siRNA were measured and visualized by super resolution microscopy (SRM) in the x, y, and z directions (FIG. 10a), and the relative fluorescence areas were plotted (FIG. 10b). When reviewing the data after the encapsulation of TNF-α siRNA, it was confirmed that in Example 2, the TNF-α siRNA labeled with green (dark gray in the drawing) FAM was positioned centrally inside the exosomes labeled with red (white in the drawing) Cy5.5. In addition, in the relative fluorescence areas, it was confirmed that Cy5.5, which showed fluorescence areas of 100 or more in Comparative Example 2 having exosomes alone, decreased to 100 or less when the TNF-α siRNA was encapsulated, and the FAM-labeled fluorescence areas relatively increased. This means that the TNF-α siRNA was not carried on the surface of the exosome by a method such as adhesion and the like, but rather, the TNF-α siRNA was precisely entered and encapsulated in the middle of the inner area of the exosome through the microscopic pores created by electroporation.

### (6) Confirmation of cellular uptake of milk-derived exosomes encapsulated with TNF-α siRNA

The pores were formed in the milk-derived exosomes by the same electroporation method as in Example 2, but the milk-derived exosomes were encapsulated with TNF-α siRNA labeled with Cy3 and incubated for three hours. Then, NCM460 cells were treated with the exosomes, and the extent of cellular uptake was visualized by a confocal microscope 24 hours later. As illustrated in FIG. 11 (dark gray indicates DPAI, light small dots indicate siRNA in the drawing), it can be seen that the introduction into the cells occurred efficiently in Example 2, where TNF-α siRNA was encapsulated in exosomes, compared to the case in which the cells were treated with TNF-α siRNA alone.

### (7) Confirmation of intracellular delivery rates of milk-derived exosomes that are encapsulated with TNF-α siRNA by electroporation

The pores were formed in the milk-derived exosomes by the same electroporation method as in Example 2, and TNF-α siRNA labeled with Cy3 was encapsulated, respectively, and stabilized at 4 °C for three hours. RAW264.7 cells were plated on confocal dishes at a density of 3 × 105 cells/dish. TNF-α siRNA was transfected using lipofectamin as a control. The RAW264.7 cells were treated with the control and Example 2, respectively, and after 24 hours, the cells were washed with DPBS, fixed in formaldehyde, and the nuclei were stained with Hoechst33342 for 10 minutes. The stained cells were photographed for relative fluorescence intensity using a confocal microscope (n=4). The fluorescence intensity of the photographed images was measured using the Image J program.

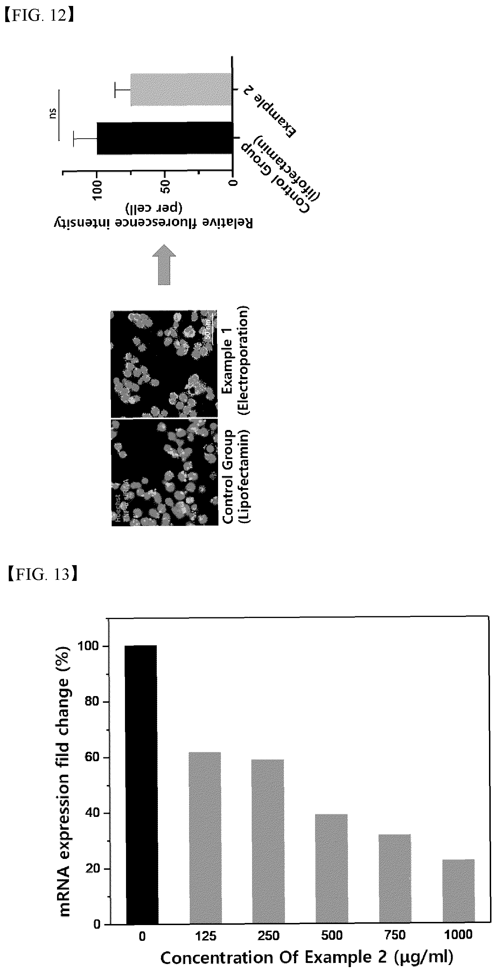

As illustrated in FIG. 12 (dark gray in the drawing indicates Hoechst, light gray indicates TNF-si-RNA), the relative TNF-α siRNA fluorescence intensity in control-treated cells was set to 100 (Lipofectamin: 100±17.19), which was set to be an intracellular delivery rate of 100 %. In contrast, the relative TNF-α siRNA fluorescence intensity in cells treated with Example 2 exhibited approximately 75 % (Example 2: 75.04±11.25). That is, it can be seen that the intracellular delivery rate of Example 2 is approximately 75 % (t-test: ns).

### (8) Confirmation of gene silencing effect of milk-derived exosomes encapsulated with TNF-α siRNA (in vitro)

The pores were formed on the milk-derived exosomes with the same condition as the electroporation method, and the milk-derived exosomes and TNF-α siRNA were mixed at a ratio of 1 mg:14 µg and incubated for 3 hours. The RAW264.7 cells preactivated with LPS (1 µg/ml, 24 hr) were treated with the milk-derived exosomes encapsulated with TNF-α siRNA (Example 2) at various concentrations. After 24 hours, TNF-α mRNA levels were measured by qRT-PCR. As a result, it was confirmed that the expression of TNF-α mRNA decreased in a concentration-dependent manner (FIG. 13).

### (9) confirmation of anti-inflammatory effects (in vivo)

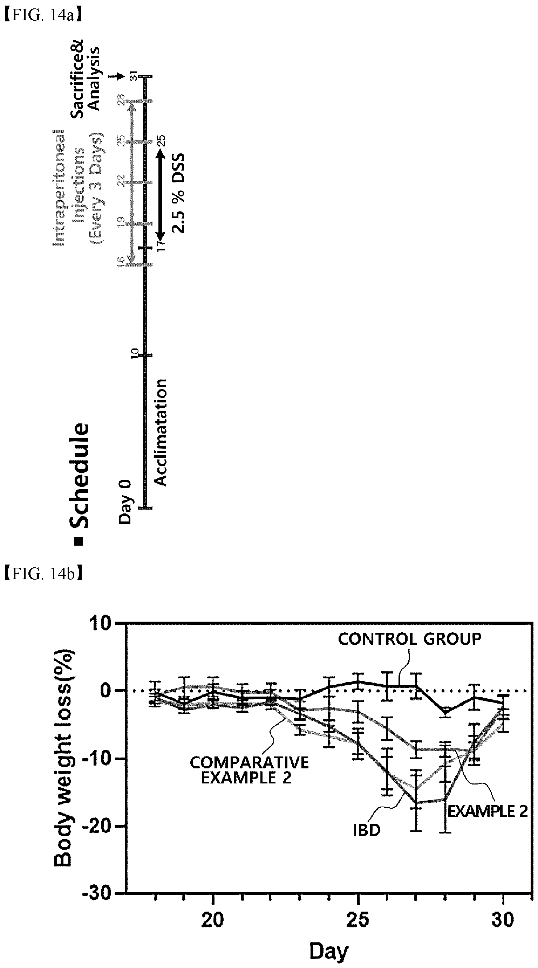

Inflammatory bowel disease (IBD) animal model (BALB/c, Female, 8w) was induced with DSS 2.5 %. The milk-derived exosomes (Comparative Example 2) and milk-derived exosomes encapsulated with TNF-α siRNA (Example 2) were injected intraperitoneally at 3-day intervals, and weight change and disease activity index (DAI) were measured (FIG. 14a). The scoring table is shown in Table 2. In addition, intestinal length and colon tissue were observed by H&E, and the H&E experimental method was the same as that of Example 1.(12).

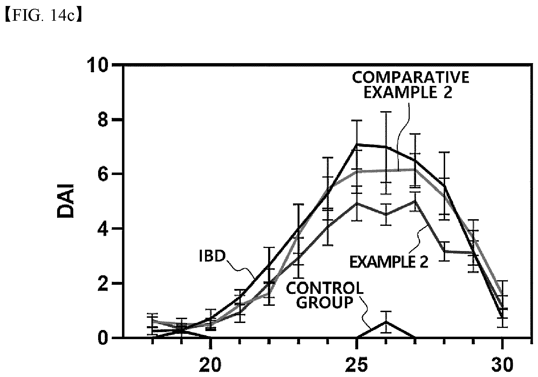

It was confirmed that intraperitoneal injection of Example 2 at 3-day intervals resulted in alleviation of weight loss, which is typical in an IBD model (FIG. 14b), and it can be seen that the milk-derived exosomes encapsulated with TNF-α siRNA have IBD inflammatory therapeutic effects (FIG. 14c).

### Embodiments

- Embodiment 1: A composition for improving intestinal health function comprising: a milk-derived exosomes; and a therapeutic agent, wherein the therapeutic agent is DNA, RNA, or a small molecule, and the therapeutic agent is positioned and encapsulated in spaces inside the exosomes.
- Embodiment 2: The composition of Embodiment 1, wherein the composition has an intracellular delivery rate of 60 to 90 %.
- Embodiment 3: The composition of any of Embodiments 1 or 2, wherein the milk is human, bovine, sheep, long-tailed goral, goat, camel, buffalo, or yak-derived milk, or colostrum.
- Embodiment 4: The composition of any of Embodiments 1 to 3, wherein the RNA is one or more RNAs selected from the group consisting of siRNA, miRNA, mRNA, shRNA, ncRNA, and antisense RNA.
- Embodiment 5: The composition of Embodiment 4, wherein the siRNA is one or more siRNAs selected from the group consisting of TNF-α siRNA, TGF-β siRNA, JAK3 siRNA, and IL-6R siRNA.
- Embodiment 6: The composition of Embodiment 4, wherein the miRNA is one or more miRNAs selected from the group consisting of Let-7b-5p, miR-320a-3p, and miR-21.
- Embodiment 7: The composition of any of Embodiments 1 to 6, wherein the composition is a liquid formulation and includes milk-derived exosomes encapsulated with the therapeutic agent at a concentration of 100 to 1000 µg/ml.
- Embodiment 8: The composition of any of Embodiments 1 to 7, wherein the composition is a lyophilized formulation and includes the milk-derived exosomes encapsulated with the therapeutic agent of 0.001 to 100 wt% based on the total weight of the composition.
- Embodiment 9: The composition of any of Embodiments 1 to 8, wherein the composition is orally administered.
- Embodiment 10: A method of manufacturing the composition of any of Embodiments 1 to 9, comprising the steps of (a) isolating exosomes from milk; (b) forming micropores on the surfaces of the exosomes by subjecting the isolated exosomes to electroporation; (c) mixing the exosomes that have undergone electroporation in step (b) with a therapeutic agent; and (d) removing the therapeutic agent that has not been encapsulated inside the exosomes.
- Embodiment 11: The method of Embodiment 10, wherein the electroporation in step (b) is to apply a voltage of 250 to 350 V.
- Embodiment 12: The method of any of Embodiments 10 to 11, wherein the electroporation in step (b) is to apply 3 to 7 pulses.
- Embodiment 13: The method of any of Embodiments 10 to 12, wherein the electroporation in step (b) is to apply a pulse for a time period of 20 to 40 ms.
- Embodiment 14: The method of any of Embodiments 10 to 13, wherein the milk in step (a) is human, bovine, sheep, long-tailed goral, goat, camel, buffalo, or yak-derived milk, or colostrum.
- Embodiment 15: The method of any of Embodiments 10 to 14, wherein the therapeutic agent in step (c) is DNA, and wherein step (d) is adding a DNase to remove the unencapsulated therapeutic agent.
- Embodiment 16: The method of any of Embodiments 10 to 15, wherein the therapeutic agent in step (c) is RNA, and wherein step (d) is adding an RNase to remove the unencapsulated therapeutic agent.
- Embodiment 17: The method of Embodiment 16, wherein the RNA is one or more RNAs selected from the group consisting of siRNA, miRNA, mRNA, shRNA, ncRNA, and antisense RNA.
- Embodiment 18: The method of Embodiment 17, wherein the siRNA is one or more siRNAs selected from the group consisting of TNF-α siRNA, TGF-β siRNA, JAK3 siRNA, and IL-6R siRNA.

Embodiment 19: The method of Embodiment 17, wherein the miRNA is one or more miRNAs selected from the group consisting of Let-7b-5p, miR-320a-3p, and miR-21.

## Claims

1. A composition for improving intestinal health function comprising:
a milk-derived exosomes; and
a therapeutic agent,
wherein the therapeutic agent is DNA, RNA, or a small molecule, and
wherein the therapeutic agent is positioned and encapsulated in spaces inside the exosomes.

2. The composition of claim 1, wherein the composition has an intracellular delivery rate of 60 to 90 %.

3. The composition of claim 1, wherein the milk is human, bovine, sheep, long-tailed goral, goat, camel, buffalo, or yak-derived milk, or colostrum.

4. The composition of claim 1, wherein the RNA is one or more RNAs selected from the group consisting of siRNA, miRNA, mRNA, shRNA, ncRNA, and antisense RNA.

5. The composition of claim 4, wherein the siRNA is one or more siRNAs selected from the group consisting of TNF-α siRNA, TGF-β siRNA, JAK3 siRNA, and IL-6R siRNA.

6. The composition of claim 4, wherein the miRNA is one or more miRNAs selected from the group consisting of Let-7b-5p, miR-320a-3p, and miR-21.

7. The composition of claim 1, wherein the composition is a liquid formulation and comprises milk-derived exosomes encapsulated with the therapeutic agent at a concentration of 100 to 1000 µg/ml.

8. The composition of claim 1, wherein the composition is a lyophilized formulation and comprises milk-derived exosomes encapsulated with the therapeutic agent of 0.001 to 100 wt% based on the total weight of the composition.

9. The composition of claim 1, wherein the composition is orally administered.

10. A method of manufacturing the composition of any one of claims 1 to 9, the method comprising the steps of:
(a) isolating exosomes from milk;
(b) forming micropores on the surfaces of the exosomes by subjecting the isolated exosomes to electroporation;
(c) mixing the exosomes that have undergone electroporation in step (b) with a therapeutic agent; and
(d) removing the therapeutic agent that has not been encapsulated inside the exosomes.

11. The method of claim 10, wherein the electroporation in step (b) is to apply a voltage of 250 to 350 V

12. The method of claim 10, wherein the electroporation in step (b) is to apply 3 to 7 pulses.

13. The method of claim 10, wherein the electroporation in step (b) is to apply a pulse for a time period of 20 to 40 ms.

14. The method of claim 10, wherein the milk in step (a) is human, bovine, sheep, long-tailed goral, goat, camel, buffalo, or yak-derived milk, or colostrum.

15. The method of claim 10, wherein the therapeutic agent in step (c) is DNA, and wherein step (d) is adding a DNase to remove the unencapsulated therapeutic agent.

16. The method of claim 10, wherein the therapeutic agent in step (c) is RNA, and wherein step (d) is adding an RNase to remove the unencapsulated therapeutic agent.

17. The method of claim 16, wherein the RNA is one or more RNAs selected from the group consisting of siRNA, miRNA, mRNA, shRNA, ncRNA, and antisense RNA.

18. The method of claim 17, wherein the siRNA is one or more siRNAs selected from the group consisting of TNF-α siRNA, TGF-β siRNA, JAK3 siRNA, and IL-6R siRNA.

19. The method of claim 17, wherein the miRNA is one or more miRNAs selected from the group consisting of Let-7b-5p, miR-320a-3p, and miR-21.
